(19) Europäisches Patentamt
European Patent Office
Office européen des brevets

(11) Veröffentlichungsnummer: **0 347 907**
**A2**

# (12) EUROPÄISCHE PATENTANMELDUNG

(21) Anmeldenummer: **89111372.2**

(51) Int. Cl.4: **A61K 31/725**

(22) Anmeldetag: **22.06.89**

(30) Priorität: **23.06.88 DE 3821271**

(43) Veröffentlichungstag der Anmeldung: **27.12.89 Patentblatt 89/52**

(84) Benannte Vertragsstaaten: **AT CH DE FR GB IT LI SE**

(71) Anmelder: **SOLCO BASEL AG**
**Rührbergstrasse 21**
**CH-4121 Birsfelden(CH)**

(72) Erfinder: **Stemberger, Axel, Dr.**
**Cramer-Klett-Strasse 35e**
**D-8014 Neubiberg(DE)**

(74) Vertreter: **Brauns, Hans-Adolf, Dr. rer. nat. et al**
**Hoffmann, Eitle & Partner, Patentanwälte**
**Arabellastrasse 4**
**D-8000 Munich 81(DE)**

## (54) Antithrombosemittel auf Basis von derivatisiertem Heparin.

(57) Antithrombosemittel, welches auf Heparin aufgebaut ist, bei dem die $SO_3H$-Gruppen ganz oder teilweise entfernt sind und bei dem weiterhin die funtionellen Gruppen, insbesondere OH-Gruppen, ganz oder teilweise verethert, verestert oder urethanisiert sind. Das erfindungsgemäße Antithrombosemittel weist keine Antikoagulationswirkung auf.

EP 0 347 907 A2

Xerox Copy Centre

## Antithrombosemittel auf Basis von derivatisiertem Heparin

Gerinnungshemmende Substanzen werden in der Medizin zur Steuerung der Hämostase bei extakorporalem Kreislauf und der Thromboseprophylaxe eingesetzt.

In der Regel wird Heparin für diese Indikationen verwendet. Heparin ist der Sammelbegriff für ein von Muscosazellen der Säugetiere synthetisiertes Gemisch saurer Polysaccharide mit einem Molekulargewicht von 3.000 bis 30.000 Dalton. Die Aufklärung der Struktur wie der Wirkung von Heparin wurde in den vergangenen Jahren intensiv von vielen Forschergruppen bearbeitet. So konnte die Struktur eines Pentasaccharids im Heparin-Molekül der blutgerinnungshemmenden Wirkung zugeordnet werden. Um im Blut gerinnungshemmend (antikoagulatorisch) zu wirken, benötigt Heparin einen plasmaständigen Cofaktor. Einer der wichtigsten Cofaktoren ist das Protein Antithrombin III. Grundlegende Arbeiten haben gezeigt, daß durch Bindung von Heparin respektive deren Pentasaccharideinheiten an basische Aminsäuren im N-terminalen Bereich von AT III die Inhibitorwirkung im Sinne einer Katalyse erheblich verstärkt wird. Intraktionen von Heparin mit der Gefäßwand sind noch nicht vollständig aufgeklärt. Diese Mechanismen sind ein weiterer Bestandteil der Wirkung von Heparin als Antithrombotikum.

Im Rahmen der sogenannten "low dose Prophylaxe" wird Heparin in geringer Dosierung appliziert, um die gefürchteten postoperativen Komplikationen, nämlich Lungenembolien und tiefe Beinvenenthrombosen, zu senken. Mit diesem Therapiekonzept konnte die Zahl der tödlichen Lungenembolien drastisch gesenkt werden. Durch die Entwicklung spezieller Heparinfraktionen konnte eine weitere Senkung der Thromboserate erzielt werden. Als Nebenwirkung der postoperativen Heparintherapie treten mit allen derzeit gängigen Präparaten postoperative Blutungen auf, mit denen Komplikationen, nämlich Blutverlust und Hämatombildung, verbunden sind.

Zur Aufklärung und zum Verständnis der Interaktion von Heparin mit den Mechanismen der Hämostase wurden nun als Grundlage für die vorliegende Erfindung gezielte Veränderungen des Heparin-Moleküls vorgenommen. Dabei stellte sich heraus, daß ein in bestimmter Weise verändertes Heparin keine blutgerinnungsverzögernde Wirkung mehr hat. Diese Aussage basiert auf in vitro-Studien mit gängigen gerinnungsanalytischen Methoden durch Nachweis der blutgerinnungsverzögernden Eigenschaften im Blut bzw. Plasma, z.B. durch Thrombelastographie, aktivierte partielle Thromboplastinzeit und der Anti-$X_a$ Aktivität. Diese Untersuchungen sind vor allem im Zusammenhang mit den Mechanismen der AT III-Wirkung von Interesse. Überraschenderweise wurde nun gefunden, daß im Körper nach Applikation der chemisch modifizierten Heparine eine dem unveränderten Heparin vergleichbare antithrombotische Aktivität nachweisbar ist. Hierzu wurden an einem etablierten Modell der kälteinduzierten Thrombose Heparin und die entsprechend der Erfindung modifizierten Präparate getestet. Völlig überraschend war es, daß gleichzeitig die bei unbehandelten, also nicht-modifizierten Heparinpräparaten ausgehende Nebenwirkung der Blutungen, also der dem normalen Heparin zuzuschreibenden, blutgerinnungshemmenden Eigenschaften, diese Wirkung bei den derivatisierten Heparinpräparaten nicht zu beobachten sind. Diese Befunde konnten nach Modifizierung der unfraktionierten wie auch der sogenannten "low molecular weight Präparate" bestätigt werden.

Aufgabe der Erfindung ist es, ein Antithrombosemittel auf Basis von Heparin zur Verfügung zu stellen, bei dem die blutgerinnungshemmenden Eigenschaften des Heparin aufgehoben sind.

Die Erfindung betrifft ein Antithrombosemittel mit verringerter Antikoagulationswirkung, das dadurch gekennzeichnet ist, daß es ein wenigstens teilweise desulfatiertes Heparin, bei dem ggf. zusätzlich die im Molekül vorhandene funktionelle Gruppen ganz oder teilweise verethert, verestert oder durch Urethanbildung entfunktionalisiert sind, enthält.

Die Desulfatierung und die ggf. vorgenommene Derivatisierung erfolgt im allgemeinen so, daß 1 bis 100 %, und vorzugsweise 20 bis 90 %, der $SO_3H$-Gruppen entfernt sind, wobei ggf. ebenfalls 1 bis 100 %, und vorzugsweise 20 bis 90 %, der funktionellen Gruppen, und zwar im wesentlichen der Hydroxylgruppen, derivatisiert sind. Geeignete Verbindungen zum Behandeln des Heparins sind insbesondere aliphatische oder aliphatisch-aromatische Mono-, Di- oder Polyhalogenide, bei denen die Halogenatome, die bevorzugt Chloratome sind, aktiv sind. Diese Reagenzien bewirken ein Verethern oder Verestern der Hydroxylgruppen. Während der Umsetzung findet gleichzeitig die Entsulfatierung des $SO_3H$-Gruppen statt.

Für die Veretherung können die für die Zuckerchemie an sich bekannten Verfahrungsbedingungen und auch die entsprechenden Reagenzien angewendet werden, wobei die Agenzien zweckmäßig so gewählt werden, daß die Derivatisierung, also Veretherung, und damit Entfunktionalisierung der Hydroxylgruppen abläuft, ohne daß durch die Veretherung wesentliche, pharmakologisch und/oder biochemisch relevante Gruppen eingeführt werden. Es genügt somit, daß man die Veretherung mit

einem kurzkettigen Alkylhalogenid, z.B. mit einem $C_{1-10}$-Alkylhalogenid, oder mit Benzylchlorid durchführt.

Das Vorgesagte gilt auch für die Veresterung an den Hydroxylgruppen. Geeignete Veresterungsmittel sind Carbonsäuremonochloride oder Dicarbonsäuredichloride, z.B. solche der Formeln

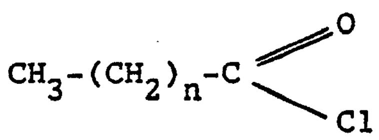

(n = 0 bis 10), wie Formylchlorid oder Acetylchlorid, oder Dicarbonsäurechloride der allgemeinen Formel

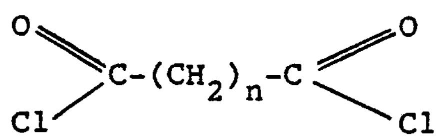

(n = 0 bis 10). Auch Carbonsäuren oder Dicarbonsäuren, die unter Veresterungsbedingungen die entsprechenden Ester bilden, können eingesetzt werden.

Schließlich kann die Derivatisierung an den Hydroxylgruppen durch Urethanbildung erfolgen. Hierzu werden als Reagenzien geeignete Mono-, Di- oder Polyisocyanate, wie sie allgemein in der Urethan-Chemie üblich sind, verwendet.

Es kann gleichzeitig eine Etherbildung, Esterbildung oder Urethanbildung durch geeignete Wahl der Reagenzien erfolgen.

Obwohl die Veretherung, Veresterung oder Urethanbildung in der Regel in wasserfreien Medien vorgenommen wird, genügen schon kleine Mengen an Feuchtigkeit, um das bei der Reaktion frei werdende Halogen unter Ausbildung einer Halogenwasserstoffsäure zu bilden, die nun wiederum die Entsulfatierung, also die Entfernung der $SO_3H$-Gruppen aus dem Molekül bewirkt.

Als Ausgangsprodukt kann hierbei Heparin, fraktioniertes Heparin, depolymerisiertes Heparin oder ein Heparinoid verwendet werden.

Das erfindungsgemäße Arzneimittel enthält ein derivatisiertes Heparin, welches in vivo die bekannte antithrombotische Wirkung aufweist, aber nur noch eine geringe oder im wesentlichen keine blutgerinnungsverhindernde Wirkung hat. Dies macht seinen Einsatz dort, wo man die Antithrombosewirkung benötigt, aber die blutgerinnungsverhindernde Wirkung unterbinden muß, besonders vorteilhaft.

Das erfindungsgemäße Antithrombosemittel kann in üblicher Weise, z.B. für die Verabreichung als Injektion, vorzugsweise für i.v. Injektionen, formuliert werden.

Beispiel

Für die nachfolgende Umsetzung wurden unfraktionierte Heparin- aber auch Heparinoidpräparate für die Humantherapie der verschiedensten Herkunft sowie auch Standard-Heparine (gemäß USP oder BP) und auch fraktionierte bzw. chemisch depolymerisierte Heparine, also die sogenannten "low molecular Präparate", verwendet. Da Heparin als polydisperser Wirkstoff standardisiert wird, ist die Umsetzung bekannter Einheiten von Heparin nach USP oder BP mit Gewichtsprozenten des Derivatisierungsagens sinnvoll.

12.500 Einheiten getrocknetes Heparin werden in 5 ml trockenem Tetrahydrofuran (THF) 3 Stunden behandelt und dann wird das THF sorgfälig durch Dekantierten entfernt. Anschließend werden 5 ml einer Lösung von 0,05 Gew.% Adipinsäuredichlorid in THF zugegeben und dieser Ansatz wird 24 Stunden bei Raumtemperatur (20 bis 22°C) inkubiert. Im Anschluß daran wird die Umsetzungslösung abdekantiert und der Rückstand wird mindestens dreimal mit frischem, trockenem THF gespült bis sich kein nicht-umgesetztes Säurechlorid mehr nachweisen läßt.

Dieses Verfahren führt im Bereich von 0,001 bis 2 Gew.% der Carbonsäuren, bezogen auf Heparin, mit den folgenden Agenzien zu erfindungsgemäßen Präparaten.

Carbonsäurechloride, wie Formylchlorid, Acetylchlorid, wobei das Carbonsäurechlorid die allgemeine Formel

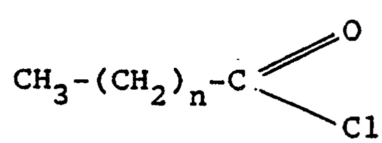

(n = 0 bis 10) oder

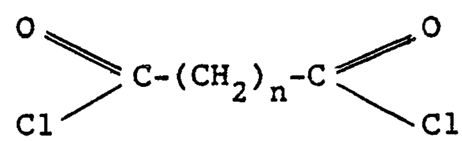

(n = 0 bis 10) hat.

## Ansprüche

1. Antithrombosemittel mit verringerter Antikoagulationswirkung, dadurch **gekennzeichnet**, daß es ein wenigstens teilweise desulfatiertes Heparin, das ggf. an den im Molekül vorhandenen funktionellen Gruppen ganz oder teilweise verethert, verestert oder durch Urethanbildung entfunktionalisiert ist, enthält.

2. Antithrombosemittel gemäß Anspruch 1, dadurch **gekennzeichnet,** daß ihm als Ausgangsprodukt Heparin, fraktioniertes Heparin, depolymerisiertes Heparin oder ein Heparinoid zugrundliegt.

3. Antithrombosemittel gemäß Ansprüchen 1 oder 2, dadurch **gekennzeichnet,** daß 1 bis 100 % der $SO_3H$-Gruppen entfernt sind und daß ggf. 1 bis 100 % der funktionellen Gruppen, und zwar im wesentlichen der OH-Gruppen, derivatisiert sind.

4. Antithrombosemittel gemäß einem der vorhergehenden Ansprüche, dadurch **gekennzeichnet,** daß das darin enthaltende Heparin als ein Gemisch vorliegt, bei dem im Heparin die $SO_3H$-Gruppen ganz oder teilweise entfernt sind und weiterhin die Hydroxylgruppen ganz oder teilweise verethert und/oder verestert und/oder mittels eines Isocyanatreagens urethanisiert sind.

5. Antithrombosemittel gemäß einem der vorhergehenden Ansprüche, dadurch **gekennzeichnet,** daß es auf Basis eines Heparins aufgebaut ist, das mittels eines Carbonsäurechlorids oder Dicarbonsäuredichlorids behandelt wurde.

6. Verwendung eines ganz oder teilweise entsulfatisierten Heparins gemäß einem der vorhergehenden Ansprüche zur Herstellung eines Arzneimittels, das als Antithrombosemittel mit verringerter Antikoagulationswirkung Verwendung findet.